(19) Europäisches Patentamt
European Patent Office
Office européen des brevets

(11) Publication number: **0 330 283 A1**

(12) **EUROPEAN PATENT APPLICATION**

(21) Application number: **89200422.7**

(22) Date of filing: **21.02.89**

(51) Int. Cl.4: **C07D 487/04 , //A61K31/40, (C07D487/04,209:00,209:00)**

(30) Priority: **25.02.88 JP 40823/88**

(43) Date of publication of application: **30.08.89 Bulletin 89/35**

(84) Designated Contracting States: **AT BE CH DE ES FR GB GR IT LI LU NL SE**

(71) Applicant: **HISAMITSU PHARMACEUTICAL CO., INC.**
**408, Tashirodaikan-machi**
**Tosu-shi Saga-ken(JP)**

(72) Inventor: **Tsuji, Masayoshi**
**992-13 Sonezakimachi**
**Tosu-shi Saga-ken(JP)**
Inventor: **Inoue, Hisataka**
**1473-10 Tsubukuhon-machi**
**Kurume-shi Fukuoka-ken(JP)**
Inventor: **Tanoue, Yoshihiro**
**1027-6 Katanawa Nakagawamachi**
**Chikushi-gun**
**Fukuoka-ken(JP)**
Inventor: **Beppu, Kouichi**
**774-1 Tashirodaikan-machi**
**Tosu-shi Saga-ken(JP)**
Inventor: **Shinohara, Ikuo**
**786-1 Tashirodaikan-machi**
**Tosu-shi Saga-ken(JP)**
Inventor: **Saita, Masaru**
**855-75 Kokura Kiyamamachi Miyaki-gun**
**Saga-ken(JP)**
Inventor: **Taniguchi, Yasuaki**
**567-1 Tashirohoka-machi**
**Tosu-shi Saga-ken(JP)**
Inventor: **Furuta, Kenichi**
**102-7 Oazaharisuri**
**Chikushino-shi Fukuoka-ken(JP)**
Inventor: **Deguchi, Yoshiki**
**774-1 Tashirodaikan-machi**
**Tosu-shi Saga-ken(JP)**
Inventor: **Hamanaka, Shoji**
**1175-3 Sakura-machi**
**Tosu-shi Saga-ken(JP)**
Inventor: **Hori, Masaaki**
**1175-3 Sakura-machi**
**Tosu-shi Saga-ken(JP)**
Inventor: **Noda, Kanji**
**320-93 Oazatsunematsu**
**Chikushino-shi Fukuoka-ken(JP)**

(74) Representative: **Timmermans, Anthonius C.Th., Ir. et al**
**European Patent Attorneys Octrooibureau Zuid P.O. Box 2287**
**NL-5600 CG Eindhoven(NL)**

(54) **Novel 5,6-diphenyl-1,2-dihydro-3H-pyrrolo[1,2-a]pyrrole-1-carboxylic acid derivatives.**

(57) Novel 5,6-diphenyl-1,2-dihydro-3H-pyrrolo[1,2-a] pyrrole-1-carboxylic acid derivatives having remarkable anti-inflammatory actions, analgesic actions and thrombocyte aggregation-inhibiting actions with remarkably less side effects such as gastroenteric tract troubles.

EP 0 330 283 A1

# NOVEL 5,6-DIPHENYL-1,2-DIHYDRO-3H-PYRROLO[1,2-a]PYRROLE-1-CARBOXYLIC ACID DERIVATIVES

## BACKGROUND OF THE INVENTION

1. Field of the Invention

This invention relates to compounds which are useful as non-steroid type antiphlogistic agents having excellent anti-inflammatory actions, analgesic actions and thrombocyte aggregation-inhibiting actions with remarkably less side effects such as gastroenteric tract troubles and more particularly it relates to novel 5,6-diphenyl-1,2-dihydro-3H-pyrrolo[1,2-a]pyrrole-1-carboxylic acid derivatives which are useful as medicines as mentioned above.

2. Description of the Prior Art

1,2-dihydro-3H-pyrrolo[1,2-a]pyrrole-1-carboxylic acid derivatives which have hitherto been reported, include those wherein a halogen atom, alkyl group, phenyl group and/or the like have been introduced to the 5-position or 6-position (Can. J. Chem., Vol. 60, pp. 2295-2312; Japanese Patent Gazette 62-54792) and those wherein an aroyl group has been introduced to the 5-position (Ger. Offen. 2731678; Japanese Pat. Appln. Laid-Open Gazette 54-24889; and Japanese Patent Gazette 62-54109). However, the compounds of the present invention as represented by the following general formula (I) wherein a phenyl group has been introduced to any one of the 5- and 6-positions, have never been disclosed and even suggested; in addition, they have never been suggested to have anti-inflammatory actions, analgesic actions and thrombocyte aggregation-inhibiting actions in any reports.

## SUMMARY OF THE INVENTION

The primary object of this invention is to provide a novel 5,6-diphenyl-1,2-dihydro-3H-pyrrolo[1,2-a]-pyrrole-1-carboxylic acid derivatives which when used will exhibit excellent anti-inflammatory actions, analgesic actions and thrombocyte aggregation-inhibiting actions with remarkably low toxicity and small side effects, such as gastroenteric tract troubles, as compared with conventional antiinflammatories and analgesics. Thus, the novel compounds are useful as highly safe medicines.

The compounds of the present invention are represented by the following general formula (I)

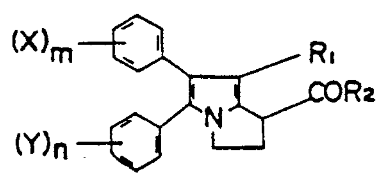

(I)

wherein m and n are each an integer of 0-3, X and Y may be identical with or different from each other and are each a halogen atom, trihalomethyl group, alkyl group, alkoxy group, alkylthio group, hydroxyl group, mercapto group, nitro group or amino group, $R_1$ is a hydrogen atom, halogen atom, alkyl group, carboxyl group or lower alkyl esters thereof, and $R_2$ is a hydroxyl group, alkoxy group or amino group.

The case wherein m and n are each zero, means (X)m and (Y)n having none of the above-mentioned substituents, that is, non-substitution.

In said general formula (I), the halogen atom is fluorine, chlorine, bromine or iodine, the trihalomethyl group is trifluoromethyl group or the like, and the alkyl group is a $C_1$-$C_6$ alkyl group such as methyl, ethyl, n-propyl, iso-propyl, n-butyl, iso-butyl or tert.-butyl group. The alkoxy group is a $C_1$-$C_6$ alkoxy group such as methoxy, ethoxy, n-propoxy, iso-propoxy, n-butoxy, iso-butoxy or tert.-butoxy group. The alkylthio group is a $C_1$-$C_6$ alkylthio group such as methylthio, ethylthio, n-propylthio, iso-propylthio, n-butylthio, iso-butylthio or tert.-butylthio group.

In said general formula (I), when X and Y are each an amino group, the amino group is a non-substituted amino group, a monoalkylamino group such as methylamino, ethylamino or propylamino group, or a dialkylamino group such as dimethylamino, diethylamino or dipropylamino group; when $R_2$ is an amino group, the amino group is a non-substituted amino group, a monoalkylamino group such as methylamino, ethylamino or propylamino group, a dialkylamino group such as dimethylamino, diethylamino or dipropylamino group, a cycloalkylamino group such as cyclobutylamino cyclopentylamino, cyclohexylamino group, a substitute alkylamino group having the formula $-NH(CH_2)_lR_3$ wherein l is an integer of 0-6, $R_3$ is hydroxyl group, an alkoxy group, a monoalkylamino group, a dialkylamino group, a cycloamino group such as piperidino, pyrrolidino, morpholino, piperazino or pipecolino, phenyl group, or a substituted phenyl group wherein a halogen atom, alkyl group, alkoxy group, nitro group, hydroxyl, trihalomethyl group or a like group has been substituted at any optional position; further, the said amino group is a heterocycloamino group such as pyridylamino, 2-(2-thiazolinyl)amino, 2-(3-methylthiazolone)imino, thienylamino, oxazolylamino, isooxazolylamino, furylamino or pyrrolylamino group, or a cycloamino group such as morpholino piperidino, pyrrolidino, piperazino or pipecolino group.

The compounds of the present invention having the above described general formula may be incorporated with a conventional vehicle to be shaped into pharmaceutically acceptable medicinal forms. They may be in the form of tablets, granules, sirups, capsules or the like for use as an oral medicine or in the form of an injection, suppository, ointment, gel, cream, lotion, liniment, plaster or the like for use as a non-oral medicine. If necessary, the compounds of the present invention may be reacted with an inorganic salt (such as a sodium salt, potassium salt, calcium salt, hydrochloride salt or aluminum salt) or with an organic salt (such as a fumarate, maleate, succinate, phthalate or lysine salt) to produce an addition salt. Further, they may be incorporated with cyclodextrin or the like to produce an inclusion compound in order to improve themselves in stability and solubility.

A process for preparing the compounds of the present invention will be illustrated hereunder. These compounds may be obtained in a good yield by the following non-limitative exemplary process:

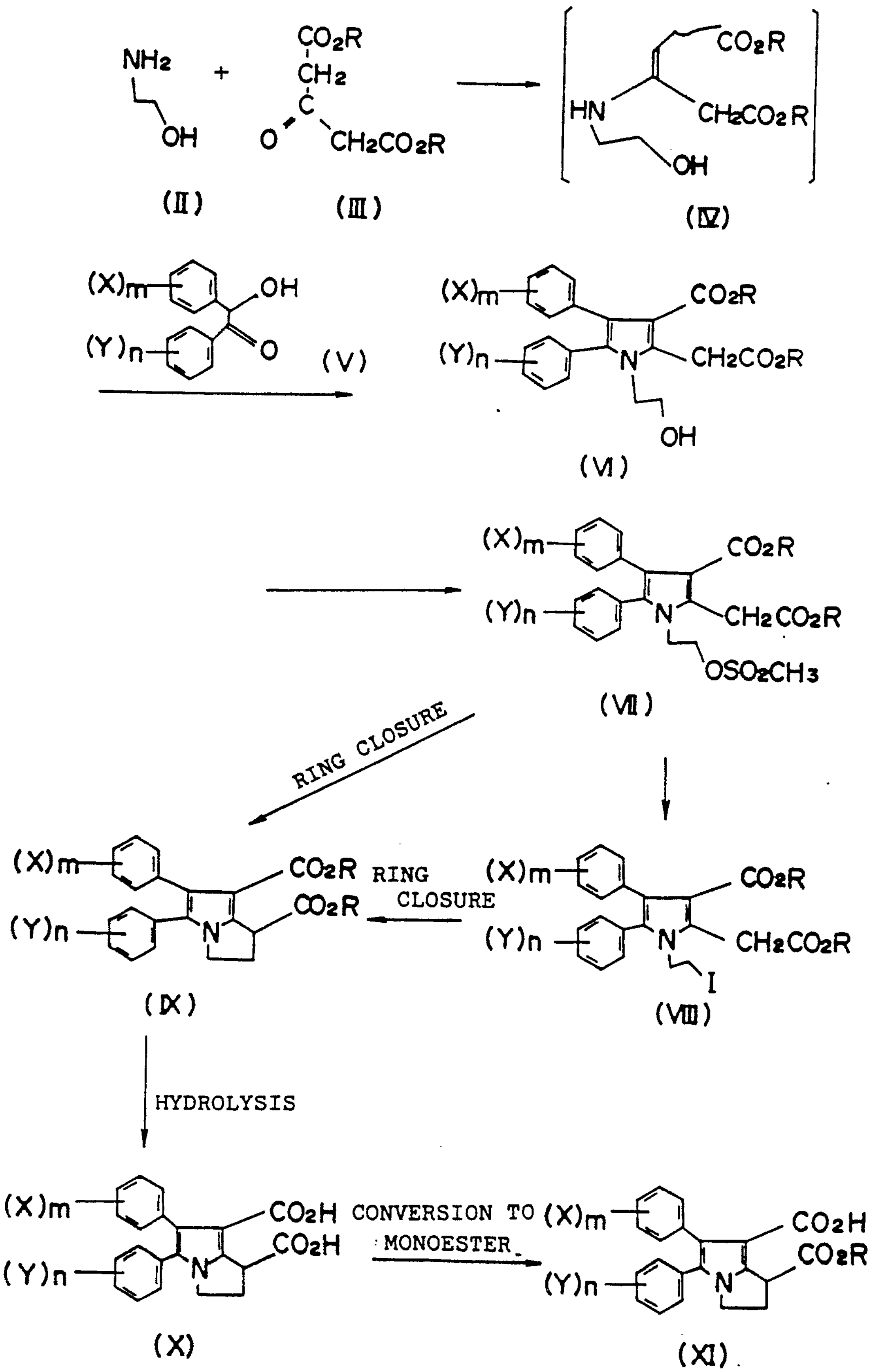


(II) (III) (IV) (V) (VI) (VII) (VIII) (IX) (X) (XI).

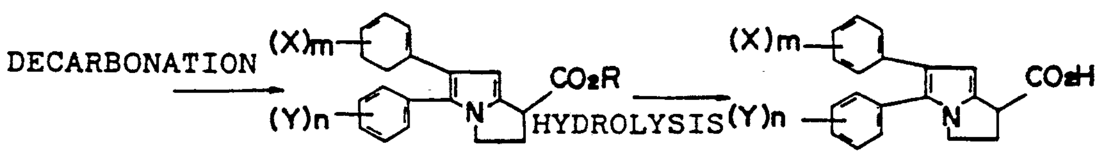


(XII) (I)

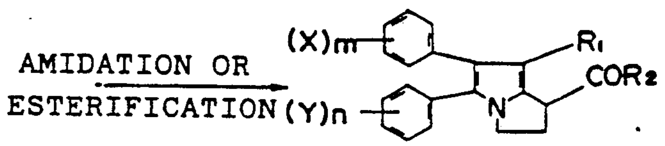


(I) ($R_1$=H, $R_2$≠OH)

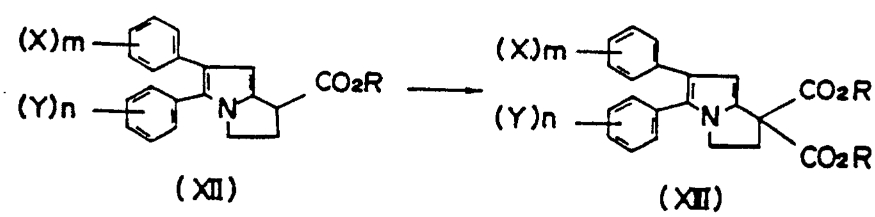

(XII) (XIII)

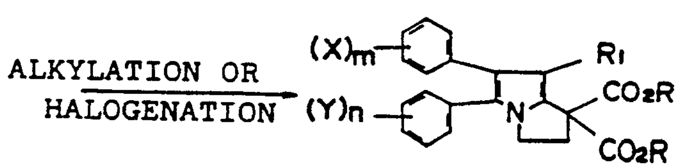


(XIV)

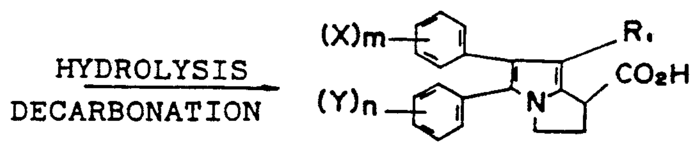


(I)

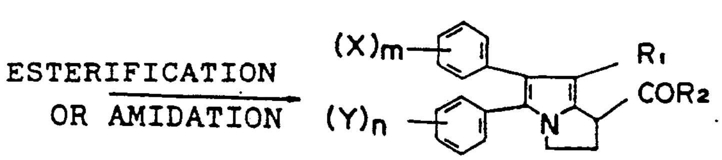


(I) ($R_2$≠OR)

In the compounds appearing in the above process, X, Y, $R_1$, $R_2$, m and n are each as defined above, and R is an alkyl group such as methyl, ethyl, n-propyl or iso-propyl group.

The above exemplary process will be concretely explained hereunder.

Monoethanolamine (II) is reacted with an acetonedicarboxylic acid diester derivative (III) to produce a compound (IV) which is then reacted with a benzoin derivative (V) in an inert solvent such as methanol or benzene and in the presence of a dehydrating catalyst such as zinc chloride, potassium bisulfate or p-toluenesulfonic acid, to obtain a compound (VI) (reference example 1). The thus obtained compound (VI) is treated with methanesulfonyl chloride in an inert solvent such as chloroform or an ether and in the presence of a tertiary amine such as triethylamine or pyridine, to obtain a mesylate compound (VII) (reference example 2). The thus obtained compound (VII) is treated with sodium iodide in an inert solvent such as acetone, acetonitrile or dimethylformamide, to obtain a compound (VIII) which is then subjected to a ring closure reaction in an inert solvent such as dimethylformamide, tetrahydrofuran or benzene and in the presence of a base such as sodium hydride, potassium carbonate, sodium methoxide, sodium ethoxide or potassium tertiary butoxide, to obtain a compound (IX). The compound (IX) may alternatively be synthesized by treating the mesylate compound (VII) in an inert solvent such as dimethylformamide or tetrahydrofuran and in the presence of a basic substance such as sodium methoxide, sodium ethoxide or sodium hydride, to effect the direct cyclization thereof. The compound (IX) is hydrolyzed in the presence of an inorganic base such as potassium carbonate or potassium hydroxide or in the presence of an inorganic acid such as hydrochloric acid or sulfuric acid, to obtain a compound (X) which is then esterified with a lower aliphatic alcohol, such as methanol, ethanol, iso-propanol or n-butanol, in the presence of hydrogen chloride thereby to obtain a compound (XI) wherein the carboxylic acid at the C-1 position has been esterified. The thus obtained compound (XI) is decarbonated under heat to produce a compound (XII) which is then hydrolyzed in the presence of an inorganic base such as potassium carbonate, potassium hydroxide, sodium hydroxide or in the presence of an inorganic acid such as hydrochloric acid or sulfuric acid, to obtain a compound (I). The compound (I) may be converted to various kinds of amides or esters. In addition, a compound (XIII) obtained by reacting the compound (XII) with a carbonate diester such as carbonate dimethyl ester, in an inert solvent such as benzene or tetrahydrofuran and in the presence of a base such as sodium hydride, is treated with a halogenating agent (such as bromine or sulfuryl chloride) to produce a compound (XIV) wherein $R_1$ is a halogen atom such as bromine or chlorine. The compound (XIII) is subjected to Friedel-Crafts reaction with an alkyl halide such as methyl iodide or ethyl iodide, in the presence of lewis acid such as aluminum chloride, to give a compound (XIV) wherein $R_1$ is an alkyl group such as methyl or ethyl group. The compound (XIV) may be hydrolyzed in the presence of an inorganic base (such as potassium carbonate or sodium hydroxide) to easily cause a decarbonating reaction thereby obtaining a compound (I) which can be converted to an ester or amide thereof in accordance with a conventional known method.

Further, the racemic compound of the formula (I) may be resolved (or subjected to mesotomism) with a suitable resolving agent in accordance with a known method to obtain individual isomers (d) and (l).

The known method is disclosed in the following publications for example:

(1) D.W. Armstrong et al: Journal of Chromatographic Science, Vol. 22 (1984) 411-415
(2) Jorgen Hermannson: Journal of Chromatography, 325 (1985) 378-384
(3) I.W. Wainer et al: Journal of Chromatography, 284 (1984) 117-124
(4) S. Allenmark et al: Journal of Chromatography, 264 (1983) 63-68
(5) S. Allenmark et al: Journal of Chromatography, 237 (1982) 473-477
(6) U.S. Patent No. 4,539,399 specification
(7) Japanese Pat. Appln. Laid-Open Gazette 60-41619
(8) T. Miwa et al: Chemical and Pharmaceutical Bulletin, Vol. 35 (1987) 682
(9) Japanese Patent Gazette 62-54109

The publication (1) discloses a resolving method using cyclodextrin, (2) discloses a technique using $\alpha_1$-acidic glycoprotein, (3) discloses a technique using (R)-N-(3,5-dinitrobenzoyl) phenylglycine, (4) and (5) disclose a resolving method using a fixed phase wherein bovine serum albumin is combined respectively with silica and agarose, (6) discloses a resolving method using a fixed phase wherein cyclodextrin is combined with silica gel, (7) discloses a resolving method using orosomucoid or material functionally analogous thereto, (8) discloses a resolving method using a fixed phase wherein ovomucoid is combined with a carrier, and (9) discloses a resolving method for decomposing with an acid by means of high-speed liquid phase chromatography, using a fixed phase combined with (1)-$\alpha$-phenylethyl alcohol.

The present invention will be better understood by the following Reference Examples and non-limitative Examples.

Reference Example 1

Twenty-six (26) grams of monoethanolamine were dissolved in 30ml of methanol, incorporated dropwise with 68g of dimethyl 1,3-acetonedicarboxylate ester at a temperature of 15 - 20°C and then agitated at room temperature for 30 minutes, after which the whole was incorporated with 100g of 4,4′-dichlorobenzoin, 58g of zinc chloride and 500ml of methanol and then refluxed under heat for 24 hours. After the end of the reaction, the resulting reaction mixture was incorporated with 45ml of a 6-N hydrochloric acid and 500ml of water and then extracted with one liter of ethyl acetate. The liquid so extracted was washed with water, dried and then freed from the solvent under a reduced pressure. The residue so obtained was recrystallized from methanol thereby to obtain 105g (64% yield) of 1-(2-hydroxyethyl)- 3-methoxycarbonyl-4,5-bis(p-chlorophenyl)pyrrole-2-methyl acetate ester having a melting point of 141-143°C represented by the general formula (VI).

Reference Example 2

Sixty (60) grams of 1-(2-hydroxyethyl)-3-methoxycarbonyl-4,5-bis(p-chlorophenyl)pyrrole-2-methyl acetate ester were dissolved in 200ml of chloroform, incorporated with 22g of triethylamine while agitating at room temperature and then incorporated dropwise with 22g of methanesulfonyl chloride so that the liquid react did not exceed 30°C in temperature. After the end of the dropwise incorporation, the resulting reaction mixture was agitated at room temperature for one hour, washed with water, a saturated aqueous solution of sodium bicarbonate and water in this order, thereafter dried and then freed from the solvent under a reduced pressure. The residue obtained was recrystallized from methanol thereby to obtain 66g (94% yield) of 1-(2-methanesulfonyloxyethyl)-3-methoxycarbonyl-4,5-bis(p-chlorophenyl)pyrrole-2-methyl acetate ester having a melting point of 99-100°C having the general formula (VII).

Example 1

Forty (40) grams of 1-(2-iodoethyl)-3-methoxycarbonyl-4,5-bis(p-chlorophenyl)pyrrole-2-methyl acetate ester were dissolved in 150ml of dimethylformamide, ice-cooled and then incorporated portionwise with 2.9g of 60% sodium hydride under agitation in a nitrogen stream. The resulting reaction mixture was agitated at room temperature for 30 minutes and then incorporated with 500ml of water to terminate the reaction. The product obtained was extracted twice with 500ml of ethyl acetate, washed with water, dried and then freed from the solvent under a reduced pressure. The residue obtained was recrystallized from isopropyl ether thereby to obtain 28g (90% yield) of 5,6-bis(p-chlorophenyl)-1,2-dihydro-3H-pyrrolo[1,2-a] pyrrole-1,7-dimethyl dicarboxylate ester.

The compound so obtained had the following melting point and elemental analysis:
Melting point 160-162°C
Elemental analysis $C_{23}H_{19}Cl_2NO_4$

| | | | |
|---|---|---|---|
| Theoretical | C 62.18 | H 4.31 | N 3.15 |
| Found | C 62.30 | H 4.29 | N 3.04 |

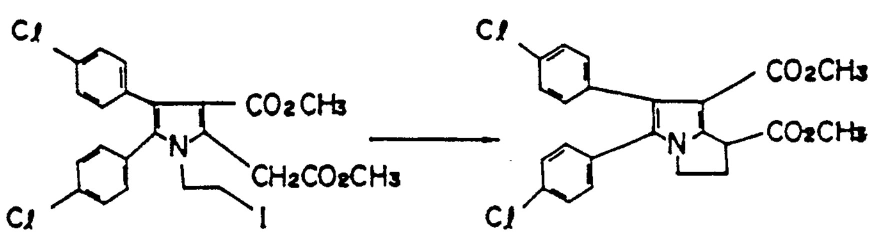

Example 2

Forty-five (45) grams of 1-(2-methanesulfonyloxyethyl)-3-methoxycarbonyl-4,5-bis(p-chlorophenyl)-pyrrole-2-methyl acetate ester were dissolved in 180ml of dimethylformamide and then incorporated little by little with 5.9g of sodium methoxide at room temperature. The resulting reaction mixture was agitated at room temperature for one hour and then poured into 500ml of water, after which the precipitate obtained was filtered off and dried. The rude crystal obtained was recrystallized from methanol thereby to obtain 32g (87% yield) of 5,6-bis(p-chlorophenyl)-1,2-dihydro-3H-pyrrolo[1,2-a]pyrrole-1,7-dimethyl dicarboxylate ester.

The melting point and elemental analysis of the compound so obtained are as follows:
Melting point 160-162°C
Elemental analysis $C_{23}H_{19}Cl_2NO_4$

| | | | |
|---|---|---|---|
| Theoretical | C 62.18 | H 4.31 | N 3.15 |
| Found | C 62.31 | H 4.32 | N 3.28 |

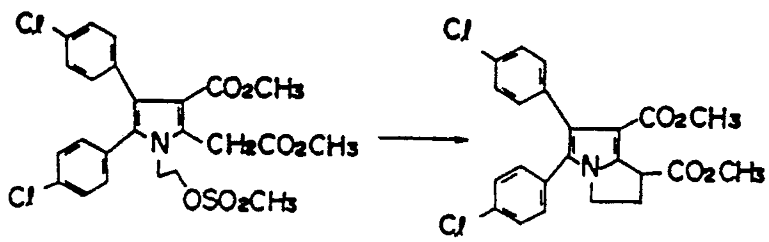

Example 3

Twenty-five (25) grams of 5,6-bis(p-chlorophenyl)-1,2-dihydro-3H-pyrrolo[1,2-a]pyrrole-1,7-dimethyl-dicarboxylate ester were dissolved in 200ml of methanol, incorporated with 200ml of an aqueous solution of 32g of potassium hydroxide and then refluxed for 6 hours. After the completion of the reaction, the resulting reaction was freed from the methanol under a reduced pressure, after which the remaining basic solution was washed with 300ml of ethyl acetate. The resulting aqueous layer was weakly acidified with conc. hydrochloric acid, extracted with 500ml of ethyl acetate, washed with water, dried and then freed from the solvent under a reduced pressure to obtain a residue. The thus obtained residue was recrystallized from ethyl acetate thereby to obtain 23g (98% yield) of 5,6-bis(p-chlorophenyl)-1,2- dihydro-3H-pyrrolo[1,2-a]-pyrrole-1,7-dicarboxylic acid.

The final compound so obtained had the following melting point and elemental analysis:
Melting point 214-217°C
Elemental analysis $C_{21}H_{15}Cl_2NO_4$

| | | | |
|---|---|---|---|
| Theoretical | C 60.59 | H 3.63 | N 3.36 |
| Found | C 60.76 | H 3.62 | N 3.41 |

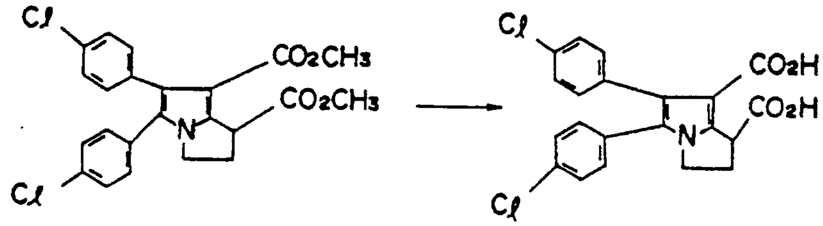

Example 4

Twenty (20) grams of 5,6-bis(p-chlorophenyl)-1,2-dihydro-3H-pyrrolo[1,2-a]pyrrole-1,7-dicarboxylic acid were introduced into 400ml of isopropyl alcohol. The whole was then saturated with hydrogen chloride gas so that the resulting reaction mixture did not exceed 10°C in temperature, followed by agitating at room temperature for two hours. After the end of the reaction, the reaction mixture obtained was poured into 1.5 liter of water to obtain a product which was filtered off, washed with water and then dried thereby to obtain 20.5g (93% yield) of 5,6-bis(p-chlorophenyl)-1,2-dihydro-3H-pyrrolo[1,2-a]pyrrole-1-isopropyl carboxylate ester-7-carboxylic acid. The compound so obtained had the following melting point and elemental analysis:
Melting point 268-272°C
Elemental analysis $C_{24}H_{21}Cl_2NO_4$

| | | | |
|---|---|---|---|
| Theoretical | C 62.89 | H 4.62 | N 3.06 |
| Found | C 62.68 | H 4.63 | N 3.10 |

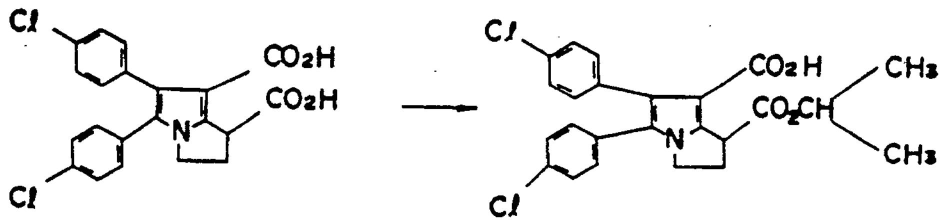

Example 5

Twenty (20) grams of 5,6-bis(p-chlorophenyl)-1,2-dihydro-3H-pyrrolo[1,2-a]pyrrole-1-isopropyl carboxylate ester-7-carboxylic acid was heated to 270-290°C for 15 minutes, allowed to cool and recrystallized from methanol thereby to obtain 15.9g (88% yield) of 5,6-bis(p-chlorophenyl)-1,2-dihydro-3H-pyrrolo[1,2-a]-pyrrole-1-isopropyl carboxylate ester.

The thus obtained compound had the following melting point and elemental analysis:
Melting point 124-126°C
Elemental analysis $C_{23}H_{21}Cl_2NO_2$

| | | | |
|---|---|---|---|
| Theoretical | C 66.67 | H 5.11 | N 3.38 |
| Found | C 66.86 | H 5.11 | N 3.43 |

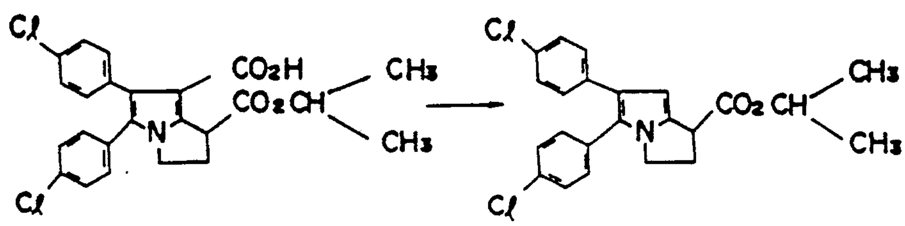

Example 6

Fifteen (15) grams of 5,6-bis(p-chlorophenyl)-1,2-dihydro-3H-pyrrolo[1,2-a] pyrrole-1-isopropyl carboxylate ester were introduced into 400ml of methanol, incorporated with 200ml of an aqueous solution of 25g

of potassium carbonate, refluxed for two hours and then concentrated by removing part of the solvent under a reduced pressure and washed with 300ml of ether, after which the aqueous layer was weakly acidified with concentrated hydrochloric acid to precipitate crystals which were filtered off. The crude crystals so filtered off were washed with water, dried and then recrystallized from ether thereby to obtain 11.5g (85% yield) of 5,6-bis(p-chlorophenyl)-1,2-dihydro-3H-pyrrolo[1,2-a]pyrrole-1-carboxylic acid.

The thus obtained compound had the following melting point and elemental analysis:
Melting point 176-179 °C
Elemental analysis $C_{20}H_{15}Cl_2NO_2$

| | | | |
|---|---|---|---|
| Theoretical | C 64.53 | H 4.06 | N 3.76 |
| Found | C 64.74 | H 4.07 | N 3.80 |

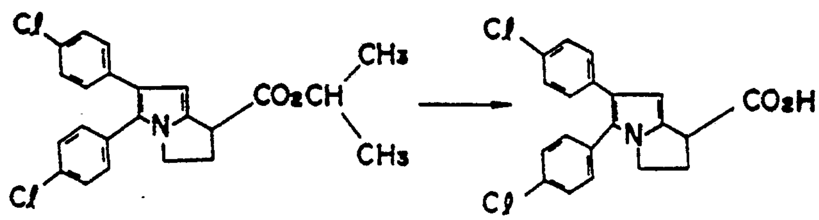

Example 7

Three (3) grams of 5,6-bis(p-chlorophenyl)-1,2-dihydro-3H-pyrrolo[1,2-a]pyrrole-1-carboxylic acid were dissolved in 20ml of tetrahydrofuran, incorporated with 0.82g of triethylamine and 0.88g of ethyl chlorocarbonate while agitating at a temperature of -5 °C, agitated at this temperature for 10 minutes and then incorporated with 0.75g of aniline, after which the whole was agitated at room temperature for 3 hours to obtain a reaction mixture. After the end of the reaction, the thus obtained reaction mixture was freed from the tetrahydrofuran under a reduced pressure, incorporated with 100ml of ethyl acetate, washed with water, dried and then freed from the solvent under a reduced pressure to obtain a residue. The residue so obtained was recrystallized from ether thereby to obtain 3.1g (86% yield) of N-phenyl-5,6-bis(p-chlorophenyl)-1,2-dihydro-3H-pyrrolo[1,2-a]pyrrole-1-carboxylic acid amide having the following melting point and elemental analysis:
Melting point 260-262 °C
Elemental analysis $C_{26}H_{20}Cl_2N_2O$

| | | | |
|---|---|---|---|
| Theoretical | C 69.81 | H 4.51 | N 6.26 |
| Found | C 70.02 | H 4.51 | N 6.21 |

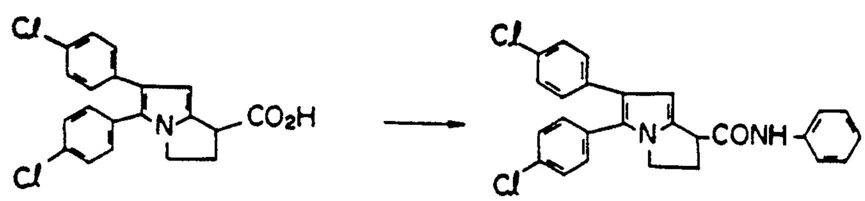

Example 8

Three (3) grams of 5,6-bis(p-chlorophenyl)-1,2-dihydro-3H-pyrrolo[1,2-a]pyrrole-1-carboxylic acid were

dissolved in 30ml of methylene chloride, cooled to a temperature between 0° and -5° C, incorporated with 1.66g of N,N′-dicyclohexylcarbodiimide and 0.59g of n-butylamine and then agitated at said temperature for 4 hours. After the end of the reaction, crystals (the N,N′-dicyclohexylcarbodiimide) precipitated in the reaction mixture were filtered out and the filtrate obtained was concentrated under a reduced pressure. The residue obtained was recrystallized from ether thereby to obtain 2.9g (84% yield) of N-bytyl-5,6-bis(p-chlorophenyl)-1,2-dihydro-3H-pyrrolo[1,2-a]pyrrole-1-carboxylic acid amide.

The thus obtained compound had the following melting point and elemental analysis:
Melting point 200-202° C
Elemental analysis $C_{24}H_{24}Cl_2N_2O$

| | | | |
|---|---|---|---|
| Theoretical | C 67.45 | H 5.66 | N 6.55 |
| Found | C 67.29 | H 5.69 | N 6.39 |

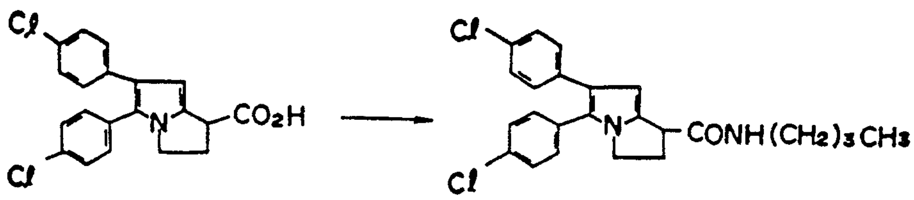

Examples 9-67

In accordance with the procedures of Examples 1-8, there were prepared the compounds having the respective general formulas indicated in the following Table 1. The symbols, (X)m, (Y)n, $R_1$ and $R_2$ in the formulas of the thus prepared compounds as well as the melting point and elemental analysis thereof are indicated in Table 1.

# Table 1

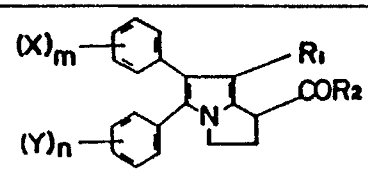

(I)

| Example No. | (X) m | (Y) n | $R_1$ | $R_2$ | Melting Point (℃) | Elemental analysis Theoretical (%) C | H | N | Found (%) C | H | N |
|---|---|---|---|---|---|---|---|---|---|---|---|
| 9 | H | H | $CO_2C_2H_5$ | $OC_2H_5$ | 111～113 | 74.42 | 6.25 | 3.47 | 74.64 | 6.26 | 3.51 |
| 10 | p-$CH_3$ | p-$CH_3$ | $CO_2CH_3$ | $OCH_3$ | 159～160.5 | 74.42 | 6.25 | 3.47 | 74.39 | 6.23 | 3.57 |
| 11 | p-F | p-F | $CO_2CH_3$ | $OCH_3$ | 165～167 | 67.15 | 4.65 | 3.40 | 67.31 | 4.64 | 3.49 |
| 12 | p-$CH_3O$ | p-$CH_3O$ | $CO_2CH_3$ | $OCH_3$ | 148～150 | 68.95 | 5.79 | 3.22 | 69.07 | 5.80 | 3.24 |
| 13 | p-$CH_3O$ | p-$CH_3O$ | $CO_2CH(CH_3)_2$ | $OCH(CH_3)_2$ | 152～154 | 70.86 | 6.77 | 2.85 | 70.99 | 6.79 | 2.80 |
| 14 | p-Cl | p-$CH_3O$ | $CO_2CH_3$ | $OCH_3$ | 147～148 | 65.53 | 5.04 | 3.18 | 65.80 | 5.05 | 3.16 |
| 15 | H | H | $CO_2H$ | OH | 210～214 | 72.61 | 4.93 | 4.03 | 72.82 | 4.93 | 4.00 |
| 16 | p-$CH_3$ | p-$CH_3$ | $CO_2H$ | OH | 172～175 | 73.58 | 5.64 | 3.73 | 73.81 | 5.63 | 3.78 |
| 17 | p-F | p-F | $CO_2H$ | OH | 251～253 | 65.80 | 3.94 | 3.65 | 65.58 | 3.93 | 3.49 |
| 18 | p-$CH_3O$ | p-$CH_3O$ | $CO_2H$ | OH | 222～225 | 67.80 | 5.20 | 3.44 | 67.55 | 5.19 | 3.40 |
| 19 | p-Cl | p-$CH_3O$ | $CO_2H$ | OH | 218～220 | 64.16 | 4.41 | 3.40 | 64.30 | 4.41 | 3.59 |
| 20 | H | H | $CO_2H$ | $OC_2H_5$ | 238～240 | 73.58 | 5.64 | 3.73 | 73.69 | 5.64 | 3.78 |

Table 1 (cont.)

| | | | | | | | | | | | |
|---|---|---|---|---|---|---|---|---|---|---|---|
| 21 | H | H | $CO_2H$ | $OCH(CH_3)_2$ | 254~ 257 | 74.02 | 5.95 | 3.60 | 74.22 | 5.95 | 3.81 |
| 22 | p-$CH_3$ | p-$CH_3$ | $CO_2H$ | $OCH(CH_3)_2$ | 275~ 280 | 74.80 | 6.52 | 3.35 | 74.68 | 6.52 | 3.33 |
| 23 | p-$Cl$ | p-$Cl$ | $CO_2H$ | $OCH_3$ | 240~ 242 | 61.41 | 3.98 | 3.26 | 61.30 | 3.98 | 3.30 |
| 24 | p-$Cl$ | p-$Cl$ | $CO_2H$ | $OC_2H_5$ | 261~ 263 | 62.18 | 4.31 | 3.15 | 62.30 | 4.30 | 3.09 |
| 25 | p-F | p-F | $CO_2H$ | $OCH(CH_3)_2$ | 268~ 270 | 67.76 | 4.98 | 3.29 | 67.99 | 4.98 | 3.38 |
| 26 | p-$CH_3O$ | p-$CH_3O$ | $CO_2H$ | $OCH(CH_3)_2$ | 269~ 272 | 69.47 | 6.05 | 3.12 | 69.68 | 6.03 | 3.24 |
| 27 | p-$Cl$ | p-$CH_3O$ | $CO_2H$ | $OCH(CH_3)_2$ | 277~ 281 | 66.15 | 5.33 | 3.09 | 66.31 | 5.34 | 3.21 |
| 28 | H | H | H | $OC_2H_5$ | 99~ 100 | 79.73 | 6.39 | 4.23 | 79.91 | 6.38 | 4.19 |
| 29 | H | H | H | $OCH(CH_3)_2$ | 102~ 104 | 79.97 | 6.71 | 4.05 | 79.79 | 6.72 | 4.19 |
| 30 | p-$Cl$ | p-$Cl$ | H | $OCH_3$ | 140~ 142 | 65.30 | 4.44 | 3.63 | 65.12 | 4.43 | 3.69 |
| 31 | p-$Cl$ | p-$Cl$ | H | $OC_2H_5$ | *1 175~ 179 0.08~0.1 mmHg | 66.01 | 4.78 | 3.50 | 66.09 | 4.78 | 3.44 |
| 32 | p-$CH_3$ | p-$CH_3$ | H | $OCH(CH_3)_2$ | 134~ 136 | 80.40 | 7.29 | 3.75 | 80.61 | 7.28 | 3.80 |
| 33 | p-F | p-F | H | $OCH(CH_3)_2$ | 139~ 141 | 72.43 | 5.55 | 3.67 | 72.64 | 5.53 | 3.60 |

*1 : Temperature of glass tube oven

Table 1 (cont.)

| | | | | | | | | | | |
|---|---|---|---|---|---|---|---|---|---|---|
| 34 | p-$CH_3O$ | p-$CH_3O$ | H | 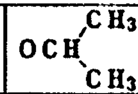 | 107~ 108 | 74.05 | 6.71 | 3.45 | 74.24 | 6.72 | 3.51 |
| 35 | p-Cl | p-$CH_3O$ | H | 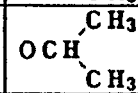 | 118~ 120 | 70.32 | 5.90 | 3.42 | 70.19 | 5.88 | 3.21 |
| 36 | H | H | H | OH | 170~ 173 | 79.19 | 5.65 | 4.62 | 79.00 | 5.63 | 4.71 |
| 37 | p-$CH_3$ | p-$CH_3$ | H | OH | 173~ 175 | 79.73 | 6.39 | 4.23 | 79.99 | 6.41 | 4.09 |
| 38 | p-F | p-F | H | OH | 170~ 172 | 70.79 | 4.46 | 4.13 | 70.59 | 4.47 | 4.28 |
| 39 | p-$CH_3O$ | p-$CH_3O$ | H | OH | 137~ 140 | 72.71 | 5.82 | 3.85 | 72.92 | 5.84 | 3.97 |
| 40 | p-Cl | p-$CH_3O$ | H | OH | 175~ 178 | 68.57 | 4.93 | 3.81 | 68.39 | 4.95 | 4.00 |
| 41 | p-Cl | p-Cl | H | 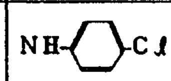 | 263~ 265 | 64.82 | 3.97 | 5.81 | 64.71 | 3.96 | 5.99 |
| 42 | p-Cl | p-Cl | H | 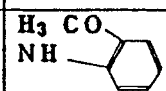 | 144~ 145 | 67.93 | 4.64 | 5.87 | 67.71 | 4.70 | 6.01 |
| 43 | p-Cl | p-Cl | H | 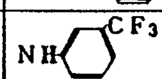 | 259~ 261 | 62.93 | 3.72 | 5.44 | 62.79 | 3.70 | 5.54 |
| 44 | p-Cl | p-Cl | H | 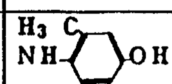 | 129~ 131 | 67.93 | 4.64 | 5.87 | 68.05 | 4.66 | 5.72 |
| 45 | p-Cl | p-Cl | H | 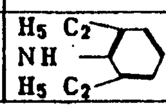 | 258~ 260 | 71.57 | 5.61 | 5.56 | 71.72 | 5.59 | 5.49 |

## Table 1 (cont.)

| | | | | | | | | | | | |
|---|---|---|---|---|---|---|---|---|---|---|---|
| 46 | p−Cl | p−Cl | H | 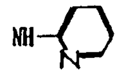 | 169~171. | 66.97 | 4.27 | 9.37 | 66.89 | 4.26 | 9.49 |
| 47 | p−Cl | p−Cl | H | 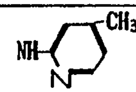 | 209~210 | 67.54 | 4.58 | 9.09 | 67.39 | 4.55 | 9.00 |
| 48 | p−Cl | p−Cl | H | 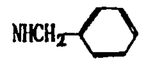 | 145~147 | 70.29 | 4.81 | 6.07 | 70.09 | 4.78 | 6.23 |
| 49 | p−Cl | p−Cl | H | 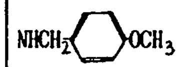 | 194~195.5 | 68.44 | 4.92 | 5.70 | 68.60 | 4.95 | 5.58 |
| 50 | p−Cl | p−Cl | H | 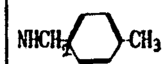 | 195~196.5 | 70.74 | 5.09 | 5.89 | 70.91 | 5.11 | 5.73 |
| 51 | p−Cl | p−Cl | H | 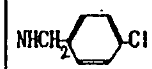 | 195.5~196.5 | 65.40 | 4.27 | 5.65 | 65.59 | 4.28 | 5.80 |
| 52 | p−Cl | p−Cl | H | 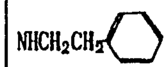 | 131~132.5 | 70.74 | 5.09 | 5.89 | 70.63 | 5.04 | 5.99 |
| 53 | p−Cl | p−Cl | H | $NH(CH_2)_3O(CH_2)_{11}CH_3$ | 119~121 | 70.34 | 7.76 | 4.69 | 70.19 | 7.75 | 4.79 |
| 54 | p−Cl | p−Cl | H | 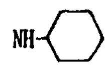 | 224~225 | 68.87 | 5.78 | 6.18 | 69.01 | 5.80 | 6.27 |
| 55 | p−Cl | p−Cl | H | $NHCH_2CH_2OH$ | 168.5~170 | 63.62 | 4.85 | 6.75 | 63.79 | 4.84 | 6.89 |
| 56 | p−Cl | p−Cl | H | 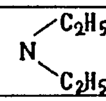 | 142.5 ~144.5 | 67.45 | 5.66 | 6.55 | 67.61 | 5.69 | 6.39 |
| 57 | p−Cl | p−Cl | H | 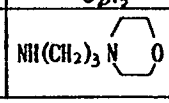 | 163~165 | 65.06 | 5.86 | 8.43 | 65.16 | 5.88 | 8.40 |

# Table 1 (cont.)

| | | | | | | | | | | | |
|---|---|---|---|---|---|---|---|---|---|---|---|
| 58 | p-Cl | p-Cl | H | 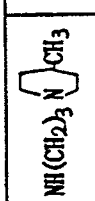 | 165~166.5 | 68.23 | 6.52 | 8.23 | 68.09 | 6.55 | 8.19 |
| 59 | p-Cl | p-Cl | H | 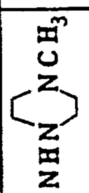 | 181~183 | 63.97 | 5.58 | 11.94 | 63.79 | 5.57 | 11.89 |
| 60 | p-Cl | p-Cl | H | 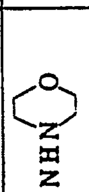 | 245~247 | 63.16 | 5.08 | 9.21 | 63.29 | 5.09 | 9.29 |
| 61 | p-Cl | p-Cl | H | 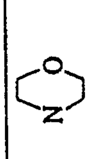 | 190.5~192 | 65.31 | 5.02 | 6.35 | 65.13 | 5.02 | 6.41 |
| 62 | H | H | H | 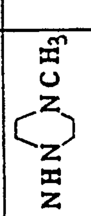 | 220~221.5 | 74.97 | 7.05 | 13.99 | 74.83 | 7.04 | 13.88 |
| 63 | p-$CH_3$ | p-$CH_3$ | H | 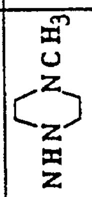 | 206~208 | 75.67 | 7.53 | 13.07 | 75.45 | 7.51 | 12.93 |
| 64 | p-F | p-F | H | 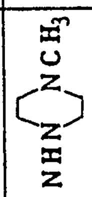 | 174~176 | 68.79 | 6.00 | 12.84 | 68.64 | 5.98 | 12.65 |
| 65 | p-F | p-F | H | $NH(CH_2)_3CH_3$ | 175~176.5 | 73.08 | 6.13 | 7.10 | 73.19 | 6.11 | 7.00 |
| 66 | p-$CH_3O$ | p-$CH_3O$ | H | 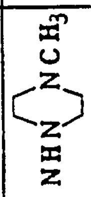 | 104~105.5 | 70.41 | 7.00 | 12.16 | 70.29 | 7.01 | 12.03 |
| 67 | p-Cl | p-$CH_3O$ | H | 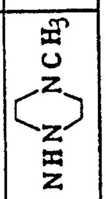 | 227~229 | 67.16 | 6.29 | 12.05 | 67.01 | 6.25 | 12.19 |

In order to substantiate the medicinal efficacy of the compounds of the present invention, methods for the pharmacological experiment of said compounds and the pharmacological data obtained by the experiment, will be indicated hereunder.

Experiment 1 Inhibitory actions on the writhing induced by acetic acid in mice

As test animals, there were provided groups consisting of 7-9 male ddY strain mice weighing 20-24g. Each of the compounds indicated in the following table was orally administered to the mice of one group. Thirty minutes later, 0.6% acetic acid was abdominally administered at a dose of 0.1ml/10g. Counting of the number of writhing was started 5 minutes later than the administration of the acetic acid and continued for 10 minutes. The inhibition rates were determined by comparing the number of writhing of the test group with that of the control group and were shown in the following Table.

Table

| Test compound | Dose (mg/kg) | Inhibition rate (%) |
|---|---|---|
| Compd. of Example 3 | 30 | 71.5 |
| Compd. of Example 4 | 30 | 56.4 |
| Compd. of Example 6 | 30 | 81.3 |
| Compd. of Example 17 | 30 | 78.4 |
| Compd. of Example 25 | 30 | 49.1 |
| Compd. of Example 37 | 30 | 45.4 |
| Compd. of Example 40 | 30 | 87.3 |

It has been found from the above results that the compounds of the present invention have remarkable inhibiting actions on the writhing induced by acetic acid.

Experiment 2 Inhibitory actions on carrageenin-induced paw edema in rats

Using, as test animals, male rats of Wistar strain each weighing approximately 150g, 0.1ml of 1% λ-carrageenin was subcutaneously injected into the paw of each rat to induce the reaction. Since then, the volume of paw edema was measured with the lapse of time. Each compound indicated in the following Table was orally administered to the rats 60 minutes before the induction of the reaction. As shown in the following Table, the results were expressed in terms of inhibition rate in comparison with those in control group 3 hours after the induction of the reaction.

Table

| Test compound | Dose (mg/kg) | Inhibition rate (%) |
|---|---|---|
| Compd. of Example 3 | 30 | 44.7 |
| Compd. of Example 4 | 30 | 31.2 |
| Compd. of Example 6 | 30 | 52.3 |
| Compd. of Example 17 | 30 | 42.9 |
| Compd. of Example 25 | 30 | 30.1 |
| Compd. of Example 37 | 15 | 27.8 |
| Compd. of Example 38 | 30 | 47.9 |
| Compd. of Example 39 | 15 | 34.7 |
| Compd. of Example 40 | 30 | 48.2 |
| Compd. of Example 7 | 30 | 37.4 |
| Compd. of Example 46 | 30 | 39.4 |
| Compd. of Example 54 | 30 | 40.2 |
| Compd. of Example 57 | 30 | 31.9 |
| Compd. of Example 58 | 30 | 36.6 |
| Compd. of Example 59 | 30 | 30.5 |

It has been found from the above results that the compounds of the present invention have significant

inhibiting actions on carrageenin-induced paw edema.

Experiment 3 Inhibitory actions on blood platelet aggregation

Nine (9) ml of blood were drawn from the concha artery of a rabbit into a syringe containing therein 1 ml of a 3.2% solution of sodium citrate, after which the whole mass was centrifuged at 1100 rpm for 10 minutes to collect platelet rich plasma (PRP). The blood from which the PRP had been collected was again centrifuged at 3000 rpm for 5 minutes to collect platelet poor plasma (PPP). The PRP was adjusted to have 500,000/μl of platelets (thrombocytes) by the use of the PPP.

Four hundred and forty-five (445)μl of the thus adjusted PRP were introduced into a cuvette, warmed to 37°C for 5 minutes, incorporated with 5μl of a solution of each of the test compounds, further warmed for two minutes and then incorporated with 50μl of collagen (10μg/ml) or 50μl of arachidonic acid (350μM) thereby to induce platelet aggregation. The platelet aggregation was measured in accordance with the Born's nephelometry by the use of an Aggregometer produced by Sysmex Co.

Table

| (Inhibiting actions on collagen aggregation) | | |
|---|---|---|
| Test compound | Final concentration (μM) | Inhibiting rate (%) |
| Compd. of Example 37 | 30 | 53.7 |
| Compd. of Example 39 | 30 | 61.7 |
| Compd. of Example 40 | 30 | 63.8 |
| Compd. of Example 7 | 10 | 78.0 |
| Compd. of Example 46 | 10 | 64.0 |
| Compd. of Example 54 | 10 | 79.5 |
| Compd. of Example 57 | 10 | 89.6 |
| Compd. of Example 58 | 10 | 83.0 |
| Compd. of Example 59 | 10 | 83.4 |

Table

(Inhibiting actions on arachidonic acid aggregation)

| Test compound | Final concentration (μM) | Inhibiting rate (%) |
|---|---|---|
| Compd. of Example 41 | 3 | 83.4 |
| | 10 | 88.7 |
| | 30 | 89.4 |
| Compd. of Example 48 | 3 | 14.3 |
| | 10 | 86.5 |
| | 30 | 91.0 |
| Compd. of Example 8 | 3 | 92.5 |
| | 10 | 90.2 |
| | 30 | 92.5 |
| Compd. of Example 54 | 3 | 93.5 |
| | 10 | 93.5 |
| | 30 | 91.9 |
| Compd. of Example 61 | 3 | 10.1 |
| | 10 | 89.4 |
| | 30 | 91.4 |

Note: Final concentration means the concentration of the test compound per 500μl of a total of the PRP (445 μl), test compound solution (5μl) and collagen (50 μl) or arachidonic acid (50μl).

It has been found that the compounds of the present invention have appreciable inhibiting actions on thrombocyte aggregation.

Experiment 4 Gastric mucous damage test in rats

Using, as test animals, male rats of Wistar strain each weighing approximately 160g, the compounds of Examples 6 and 40 as well as indomethacin were each orally administered to the rats at the dose indicated in the following Table after their fasting for 18 hours. Three and a half (3.5) hours later, the rats were dissected to remove the stomachs therefrom. 10ml of a 70% ethanol were injected into the stomachs to fix them, and the stomachs were cut and washed with water and then visually observed to find whether ulcers were formed therein.

Table

| Test compound | Dose (mg/kg) | Ulcer formation (cases out of 7) |
|---|---|---|
| Compd. of Example 6 | 100 | 0/7 |
| Compd. of Example 40 | 100 | 0/7 |
| Indomethacin | 10 | 7/7 |

It has been found from the above results that the compounds of the present invention will not cause gastric mucous membrane disturbances as side effects when administered.

[Effects of the Present Invention]

As is so far mentioned, 5,6-diphenyl-1,2-dihydro-3H-pyrrolo[1,2-a]pyrrole-1-carboxylic acid derivatives are those which were first synthesized by the present inventors and have a novel chemical structure. As is apparent from the above experiments, etc., they have remarkable anti-inflammatory actions, analgesic actions and thrombocyte aggregation-inhibiting actions with remarkably less side effects such as gastroenteric tract troubles. Accordingly, they are very industrially useful as anti-inflammatories, analgesics or thrombocyte aggregation-inhibiting medicines.

**Claims**

1. A 5,6-diphenyl-1,2-dihydro-3H-pyrrolo[1,2-a] pyrrole-1-carboxylic acid derivative represented by the general formula (I)

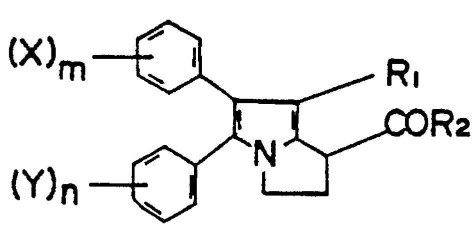 .

(I)

wherein m and n are each an integer of 0-3, X and Y are identical with, or different from, each other and are each a halogen atom, trihalomethyl group, alkyl group, alkoxy group, alkylthio group, hydroxyl group, mercapto group, nitro group or amino group, $R_1$ is a hydrogen atom, halogen atom, alkyl group, carboxyl group or lower alkyl ester thereof, and $R_2$ is a hydroxyl group, an alkoxy group or amino group.

European Patent Office

# EUROPEAN SEARCH REPORT

Application number

EP 89200422.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A1 - 0 041 711 (SYNTEX)<br>* Abstract; claims *<br>-- | 1 | C 07 D 487/04<br>//A 61 K 31/40<br>(C 07 D 487/04<br>C 07 D 209:00<br>C 07 D 209:00) |
| A | EP - A2/A3 - 0 053 021 (SYNTEX)<br>* Abstract; claims *<br>-- | 1 | |
| D,A | CANADIAN JOURNAL OF CHEMISTRY, vol. 60, no. 17, 1982<br>H. CARPIO, J. ACKRELL et al. "Synthesis of 1,2-dihydro-3H-pyrrolo[1,2-a]pyrrole-1-carboxylic acids and homologous pyridine and azépine analogues thereof"<br>pages 2295-2312<br>* Pages 2297-2301; compounds 11d,e,f, 12b,c *<br>---- | 1 | TECHNICAL FIELDS SEARCHED (Int. Cl.4)<br>C 07 D 487/00 |

The present search report has been drawn up for all claims

| Place of search | Date of completion of the search | Examiner |
|---|---|---|
| VIENNA | 18-04-1989 | JANISCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document